# EUROPÄISCHE PATENTSCHRIFT

(11) **EP 3 019 026 B1**
(45) Veröffentlichungstag und Bekanntmachung des Hinweises auf die Patenterteilung: **20.09.2017**
(21) Anmeldenummer: 14736377.4
(22) Anmeldetag: 03.07.2014
(51) Int. Cl.: A23L 2/00, A61K 8/73, A61Q 19/00, A61Q 19/08, C02F 1/68

(54) **VERFAHREN UND VORRICHTUNG ZUR BEREITSTELLUNG EINER HYALURONSÄURE ENTHALTENDEN ZUSAMMENSETZUNG**
METHOD AND DEVICE FOR PROVIDING A COMPOSITION CONTAINING HYALURONIC ACID
PROCÉDÉ ET DISPOSITIF POUR FOURNIR UNE COMPOSITION CONTENANT DE L'ACIDE HYALURONIQUE

(30) Priorität: 08.07.2013 DE 102013213335
(43) Veröffentlichungstag der Anmeldung: 18.05.2016
(73) Patentinhaber: BWT Aktiengesellschaft, 5310 Mondsee (AT)
(72) Erfinder: JOHANN, Jürgen, 69226 Nussloch (DE)
(74) Vertreter: Pfiz, Thomas
(86) Internationale Anmeldenummer: PCT/EP2014/064188
(87) Internationale Veröffentlichungsnummer: WO 2015/003995

(56) Entgegenhaltungen:
- EP-A1- 2 532 252
- EP-A2- 1 498 392
- WO-A1-2006/070453
- CN-A- 103 006 475
- DE-A1-102009 001 343
- DE-A1-102012 108 227
- DE-U1-202005 015 270
- JP-A- 2003 277 218
- US-A1- 2002 017 494
- US-A1- 2008 020 096
- Www.Inga-Ro-Systems.Eu: "Umkehrosmose Wasseraufbereitung nach modernsten Standards", , 13. Juni 2013 (2013-06-13), XP055132878, Gefunden im Internet: URL:https://web.archive.org/web/2013061304 1434/http://www.inga-ro-systems.eu/ [gefunden am 2014-08-01]

## Beschreibung

Die Erfindung betrifft ein Verfahren und eine Vorrichtung zur Bereitstellung einer Hyaluronsäure enthaltenden Zusammensetzung insbesondere für kosmetische oder medizinische Zwecke.

Hyaluronsäure ist chemisch gesehen Glykosaminoglykan. Aufgebaut ist die Hyaluronsäure, wie alle Glykosaminoklykane, als makromolekulare Kette aus sich wiederholende Zuckereinheiten (Disaccharide). Dabei können sich bis zu 100.000 solcher Bausteine hintereinanderreihen und so die klassische Hyaluronverbindung ausbilden. Wird das Molekül hydratisiert (das bedeutet, es kommt mit Wasser in Verbindung) dehnt es sich aus und nimmt bis zu 10.000 mal mehr Raum ein als in seinem Grundzustand. So kann zum Beispiel ein Gramm Hyaluronsäure bis zu 6 Liter Wasser speichern. Sichtbar wird diese Ausdehnung durch ein gelartiges Erscheinungsbild. Hyaluronsäure ist also ein ausgezeichneter Wasserbinder.

Hyaluronsäure-Moleküle, die lang und groß sind und eine hohe Viskosität (Schmierung) bzw. Druckbeständigkeit aufweisen, ermöglichen es den Gelenken sowie der Haut das Gewicht zu tragen bzw. Spannungen zu widerstehen. Besonders große Anteile an Hyaluronsäure findet man vor allem im Gelenkknorpel, im Glaskörper des Auges und in vielen Geweben des Körpers, die vor allem eine stabilisierende Aufgabe haben. Je nachdem in welcher Ausdehnung die Hyaluronsäure vorliegt kann neben einem weichen Zustand auch ein eher zäher hartgummiähnlicher Zustand erreicht werden, der die Eigenschaft hat, dass großer Druck abgefedert wird. Die Struktur der Hyaluronsäure hat des Weiteren eine formende und richtungsweisen Eigenschaft, d.h. an den meisten Stellen des Körpers, wo es wichtig ist, dass ein bestimmter Halt oder eine Form beibehalten wird, kommt die Hyaluronsäure vor. Letztendlich ist in den meisten menschlichen Zellen Hyaluronsäure vorhanden und sorgt für die Struktur und die Stabilität der Zellwand.

Hyaluronsäure ist auch ein natürlicher Bestandteil der Haut. Sie bindet effektiv Feuchtigkeit in der oberen Hautschicht. Ihre feuchtigkeitsbindende Wirkung ergibt sich daraus, dass sie ihren Hydratmantel erst nach und nach abgibt und auf diese Weise über einen langen Zeitraum auf der Haut wirkt. Ihr optisch hautstraffender und glättender Effekt resultiert aus der Verdunstung von Wasser und der dadurch bedingten leichten Straffung des Gelfilms auf der Haut. Die Produktion von körpereigener Hyaluronsäure nimmt mit zunehmendem Alter ab; dadurch kommt es zu Hautalterungserscheinungen, verstärkte Faltenbildung, trockener Haut und Elastizitätsverlust.

In diesem Zusammenhang ist es in der Kosmetikindustrie bekannt, Hyaluronsäure in Cremes oder Gelen einzusetzen. D.h. die Hyaluronsäure wird in Kombination mit vielen anderen Inhaltstoffen, wie zum Beispiel Öle, Fette, Stearinsäure bzw. Stearine, Glycerin, Silicate etc. formuliert. Das Einmassieren der Cremes in die Haut erfordert einen gewissen Aufwand und hinterlässt auch fettige Rückstände der Trägersubstanz.

Ausgehend hiervon liegt der Erfindung die Aufgabe zugrunde, die im Stand der Technik bekannten Verfahren und Vorrichtungen zur Herstellung bzw. Bereitstellung von Hyaluronsäure insbesondere für kosmetische und medizinische Zwecke weiter zu verbessern und dabei eine effektive und einfache Anwendung zu ermöglichen.

Zur Lösung dieser Aufgabe wird die im Patentanspruch 1 bzw. 8 angegebene Merkmalskombination vorgeschlagen. Vorteilhafte Ausgestaltungen und Weiterbildungen der Erfindung ergeben sich aus den abhängigen Ansprüchen. Die Erfindung geht von dem Gedanken aus, durch Zugabe von Hyaluronsäure ins Trinkwasser eine natürliche Aufnahme insbesondere auf der Haut zu ermöglichen. Demzufolge wird in verfahrensmäßiger Hinsicht vorgeschlagen, dass Hyaluronsäure in einer Dosiervorrichtung bevorratet wird, und dass aus einem Trinkwassernetz entnommenes Trinkwasser mittels der Dosiervorrichtung unter Bildung der Zusammensetzung mit Hyaluronsäure versetzt wird. Damit kann die Hyaluronsäure sehr einfach und mit großer Wirkung, mit dem Trinkwasser frei von Rückständen in der Haut aufgenommen werden. Das Wasser weicht die Haut auf, sie wird leichter durchlässig und dadurch gelangt Hyaluronsäure besser in die Haut hinein. Der Effekt der Filmbildung auf der Haut und die Glättung der Haut ist direkt sichtbar und feststellbar. So kann zum Beispiel beim Duschen, Hyaluronsäure ins Duschwasser dosiert werden, wobei die Dosierung zweckmäßig nach dem Abseifen erfolgt. Die Hyaluronsäure wird mit dem Wasser über die Haut aufgenommen und bildet einen angenehm wirkenden Film auf der Haut. Die Dosierung von Hyaluronsäure in das aus dem Trinkwassernetz entnommene Dusch- bzw. Brauchwasser erspart somit das aufwendige Cremen nach dem Duschen. Die Anwendung ist einfach und nur Hyaluronsäure wird von der Haut aufgenommen. Insbesondere beim Duschen mit Wassertemperaturen von üblicherweise mehr als 30 °C dringt die Hyaluronsäure besser in die Haut ein.

Die Bereitstellung von Hyaluronsäure im Trinkwasser ist aber nicht nur auf äußerliche Anwendungen beschränkt, sondern umfasst auch die orale Aufnahme zur Versorgung der Körpermatrix. Auch hierfür müssen keine zusätzlichen Inhaltsstoffe formuliert werden.

Der Dosiervorgang lässt sich dadurch besonders einfach gestalten, dass das Trinkwasser unter Aufnahme von Hyaluronsäure strömend durch die Dosiervorrichtung hindurchgeleitet wird.

Eine Handhabungserleichterung ergibt sich dadurch, dass die aus Trinkwasser und Hyaluronsäure gebildete Flüssigzusammensetzung über eine Wasserauslaufarmatur insbesondere an einer Dusche, Badewanne oder einem Waschbecken zur Anwendung auf der Haut ausgegeben wird.

Um die orale Verabreichung zu vereinfachen, ist es vorteilhaft, wenn das Trinkwasser in einen die Dosiervorrichtung enthaltenden Behälter zur Bereitung von Getränken eingefüllt wird.

Vorteilhafterweise wird die Hyaluronsäure in einer Konzentration im Bereich von 2 bis 2000 mg/l in das Trinkwasser eindosiert.

Weitere vorteilhafte Ausgestaltungen sehen vor, dass niedermolekulare Hyaluronsäure mit einem Molekulargewicht < 400 000 Dalton oder hochmolekulare Hyaluronsäure mit einem Molekulargewicht größer 1 000 000 Dalton in das Trinkwasser eindosiert wird. Für die äußerliche Filmbildung wird üblicherweise hochmolekulare Hyaluronsäure verwendet, während die Dosierung von niedermolekularer Hyaluronsäure eine Penetration durch die Epidermis erleichtert, so dass die Hyaluronsäure in der Dermis gespeichert werden kann.

Um eine Keimbildung in der Dosiervorrichtung zu vermeiden, ist es vorteilhat, wenn die Hyaluronsäure mit einem Konservierungsmittel, insbesondere Sorbinsäure oder Silberharz versetzt wird. Dabei kann die Sorbinsäure in Hyaluronsäure insbesondere als Pulver oder Tablette gemischt bzw. eingebracht werden, wobei die Sorbinsäurekonzentration etwa 0,2 % betragen sollte. Zur Konservierung mit Silber kann Silberharz mit Hyaluronsäure gemischt werden, mit einer Konzentration von etwa 1 % Silberharz.

Im Hinblick auf eine Vorrichtung zur Bereitstellung einer Hyaluronsäure enthaltenden Zusammensetzung wird die eingangs genannte Aufgabe durch eine Hyaluronsäure enthaltende Dosiervorrichtung gelöst, die mit Trinkwasser aus einem Trinkwassernetz beaufschlagbar ist, wobei die Zusammensetzung aus Trinkwasser und Hyaluronsäure gebildet wird. Dabei ergeben sich die bereits im Zusammenhang mit dem Verfahren genannten vorteilhaften Wirkungen gleichermaßen. Analoge Merkmale und Maßnahmen sind daher auch entsprechend kombinierbar.

Um den baulichen Aufwand zu reduzieren und die Handhabung zu erleichtern, ist es vorteilhaft, wenn die Dosiervorrichtung einen Druckanschluss zum Einleiten von unter Druck stehendem Trinkwasser aufweist.

Um auch den Wartungsaufwand zu verringern, ist es von Vorteil, wenn die Dosiervorrichtung eine Hyaluronsäure enthaltende Wechselkartusche aufweist.

Eine ungewollte Rückströmung lässt sich dadurch vermeiden, dass im Zulauf und Ablauf der Wechselkartusche ein Rückschlagventil angeordnet ist.

Für eine gezielte Einhaltung von Dosiermengen ist es vorteilhaft, wenn die Dosiervorrichtung eine Dosierpumpe zur Flüssigdosierung oder eine Dosierschleuse zur Feststoffdosierung aufweist.

Weitere Gebrauchsvorteile ergeben sich dadurch, dass die Dosiervorrichtung über ein Umschaltventil bedarfsweise zur Lieferung der Zusammensetzung oder von Trinkwasser umschaltbar ist.

Gegenstand der Erfindung ist auch die Verwendung einer Zusammensetzung aus Trinkwasser und Hyaluronsäure zur Hautbehandlung.

Im Folgenden wird die Erfindung anhand der in der Zeichnung in schematischer Weise dargestellten Ausführungsbeispiele näher erläutert. Es zeigen
- Fig. 1: ein Blockschaltbild einer Vorrichtung zur Bereitstellung von Hyaluronsäure zur Hautbehandlung;
- Fig. 2: eine schematisch vereinfachte Darstellung einer Vorrichtung zur Bereitstellung von Hyaluronsäure in einem Trinkwasserkrug;
- Fig. 3a und b: ein Blockschaltbild einer Hyaluronsäure enthaltenden Dosiervorrichtung in einer Sperrstellung und einer Bedarfstellung.

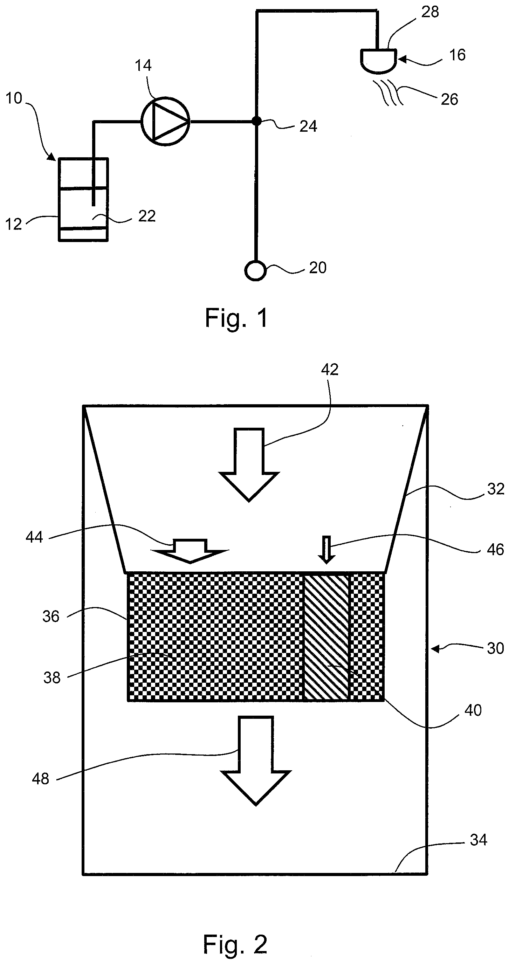

Die in Fig. 1 dargestellte Vorrichtung umfasst eine Dosiereinrichtung 10 mit Vorratsbehälter 12 und Dosierpumpe 14 sowie eine Dusche 16 mit Duschkopf 18 und Druckanschluss 20 für Trinkwasser. Der Vorratsbehälter 12 enthält eine wässrige Hyaluronsäurelösung 22, die mittels der Dosierpumpe 14 an der Abzweigstelle 24 in das gezapfte Trinkwasser eindosierbar ist. Die Zusammensetzung aus Trinkwasser und Hyaluronsäure 26 lässt sich dann nach Bedarf über den Duschkopf 28 beim Duschen auf die Haut applizieren.

Die Dosiereinrichtung nach Fig. 2 weist einen Wasserkrug (Pitcher) 30 auf, der einen oberen Trichterteil 32, einen bodenseitigen Sammelteil 34 und eine Filterkartusche 36 am Auslauf des Trichterteils 32 umfasst. Die Filterkartusche 36 enthält eine Schüttung 38 aus einem Filtermaterial und einen Kartuscheneinsatz 40 mit Hyaluronsäure als Feststoff.

Das aus einem nicht gezeigten Trinkwassernetz gezapfte Trinkwasser 42 wird in den Trichterteil 32 eingefüllt, wobei unter Schwerkrafteinfluss ein Hauptstrom 44 durch das Filtermaterial 38 beispielsweise entkalkt wird, während ein Teilstrom 46 durch den Kartuscheneinsatz 40 gelangt und dabei mit Hyaluronsäure angereichert wird. Die Zusammensetzung 48 aus gefiltertem Trinkwasser und Hyaluronsäure wird dann in dem Bodenteil 34 gesammelt und kann von dort bei Bedarf über einen nicht gezeigten Auslauf in einen Trinkbehälter gefüllt werden.

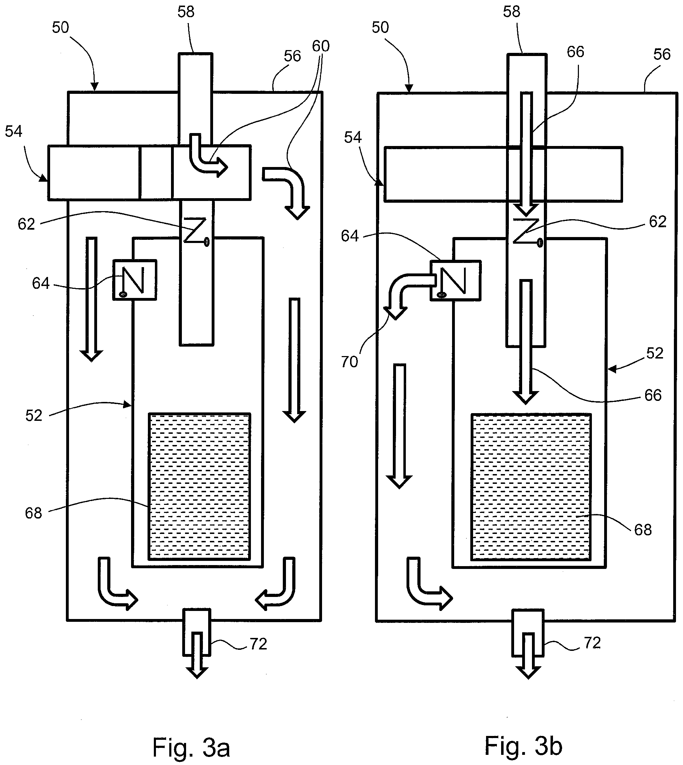

Fig. 3a und 3b zeigt eine weitere Ausführungsform einer Dosiervorrichtung 50 mit einer Dosierschleuse 52 für Hyaluronsäure und einem Umschaltventil 54 in einem Aufnahmebehälter 56 zur bedarfsgerechten Eindosierung der Hyaluronsäure.

In der in Fig. 3a gezeigten Schaltstellung des Umschaltventils 54 wird über den Trinkwasseranschluss 58 nur der Aufnahmebehälter 56 in Richtung der Strömungspfeile 60 mit Druckwasser beaufschlagt, während die Dosierschleuse 52 über Rückschlagventile 62,64 am Ein- und Auslass abgesperrt bleibt. Das Rückschlagventil 62 verhindert dabei eine Rückströmung über den Einlass in die Trinkwasserströmung 60, und das Rückschlagventil 64 sperrt den Auslass gegen Eindringen von Trinkwasser aus dem umgebenden Behälterraum ab.

In der in Fig. 3b gezeigten Schaltstellung des Umschaltventils 54 wird der Trinkwasseranschluss 58 mit dem Innenraum der Dosierschleuse 52 verbunden, wobei das Trinkwasser in Richtung der Strömungspfeile 66 durch das Rückschlagventil 62 hindurch einströmt und in Kontakt mit der Feststoffschüttung 68 aus Hyaluronsäure gelangt. Die Zusammensetzung aus Trinkwasser und Hyaluronsäure strömt dann aus der Dosierschleuse 52 über Rückschlagventil 64 in den Aufnahmebehälter (Strömungspfeil 70) und kann dann am Behälterausgang 72 gezapft werden. Die Dosiervorrichtung 50 kann in der Zuleitung einer Wasserauslaufarmatur, z.B. einer Dusche eingebaut werden, wobei die Feststoffschüttung 68 zweckmäßig durch eine Wechselkartusche gebildet ist.

## Patentansprüche

1. Verfahren zur Bereitstellung einer Hyaluronsäure enthaltenden Zusammensetzung insbesondere für kosmetische oder medizinische Zwecke, bei welchem Hyaluronsäure in einer Dosiervorrichtung (10,30,50) bevorratet wird, **dadurch gekennzeichnet, dass** aus einem Trinkwassernetz (20) entnommenes, unter Druck stehendes Trinkwasser über einen Druckanschluss (58) in die Dosiervorrichtung (10,30,50) strömend eingeleitet und mittels der Dosiervorrichtung (10,30,50) unter Bildung der Zusammensetzung mit Hyaluronsäure versetzt wird, und dass die aus Trinkwasser und Hyaluronsäure gebildete Zusammensetzung über einen Duschkopf (28) einer Dusche (16) zur Anwendung auf der Haut ausgegeben wird.

2. Verfahren nach Anspruch 1, **dadurch gekennzeichnet, dass** das Trinkwasser unter Aufnahme von Hyaluronsäure strömend durch die Dosiervorrichtung (10,30,50) hindurchgeleitet wird.

3. Verfahren nach Anspruch 1 oder 2, **dadurch gekennzeichnet, dass** Hyaluronsäure in einer Konzentration im Bereich von 2 bis 2000 mg/l in das Trinkwasser eindosiert wird.

4. Verfahren nach einem der Ansprüche 1 bis 3, **dadurch gekennzeichnet, dass** niedermolekulare Hyaluronsäure mit einem Molekulargewicht < 400 000 Dalton oder hochmolekulare Hyaluronsäure mit einem Molekulargewicht größer 1 000 000 Dalton in das Trinkwasser eindosiert wird.

5. Verfahren nach einem der Ansprüche 1 bis 4, **dadurch gekennzeichnet, dass** die Hyaluronsäure mit einem Konservierungsmittel, insbesondere Sorbinsäure versetzt wird.

6. Vorrichtung zur Bereitstellung einer Hyaluronsäure enthaltenden Zusammensetzung insbesondere für kosmetische oder medizinische Zwecke, umfassend eine Hyaluronsäure enthaltende Dosiervorrichtung (10,30,50), **dadurch gekennzeichnet, dass** die Dosiervorrichtung (10,30,50) einen Druckanschluss (58) zum Einleiten von unter Druck stehendem Trinkwasser aus einem Trinkwassernetz (20) aufweist, wobei mittels der Dosiervorrichtung (10,30,50) die Zusammensetzung aus Trinkwasser und Hyaluronsäure gebildet und über einen Duschkopf (28) einer Dusche (16) zur Anwendung auf der Haut ausgegeben wird.

7. Vorrichtung nach Anspruch 6, **dadurch gekennzeichnet, dass** die Dosiervorrichtung (10,30,50) eine Hyaluronsäure enthaltende Wechselkartusche (68) aufweist.

8. Vorrichtung nach Anspruch 7, **dadurch gekennzeichnet, dass** im Zulauf und Ablauf der Wechselkartusche (68) ein Rückschlagventil (62,64) angeordnet ist.

9. Vorrichtung nach einem der Ansprüche 6 bis 8, **dadurch gekennzeichnet, dass** die Dosiervorrichtung (10,30,50) eine Dosierpumpe (14) zur Flüssigdosierung oder eine Dosierschleuse (52) zur Feststoffdosierung aufweist.

10. Vorrichtung nach einem der Ansprüche 6 bis 9, **dadurch gekennzeichnet, dass** die Dosiervorrichtung (10,30,50) über ein Umschaltventil (54) bedarfsweise zur Lieferung der Zusammensetzung oder von Trinkwasser umschaltbar ist.

## Claims

1. A method for providing a composition containing hyaluronic acid, in particular for cosmetic or medical purposes, in which hyaluronic acid is provided in a metering device (10, 30, 50), **characterized in that** potable water taken from a potable water network (20) under pressure is introduced by a pressure connection (58) to stream into the metering device (10, 30, 50) and is admixed with hyaluronic acid by means of the metering device (10, 30, 50) with formation of the composition, and that the composition formed from potable water and hyaluronic acid is discharged via a showerhead (28) of a shower (16) for use on the skin.

2. The method as claimed in claim 1, **characterized in that** the potable water is passed as a flow through the metering device (10, 30, 50) with uptake of hyaluronic acid.

3. The method as claimed in claim 1 or 2, **characterized in that** hyaluronic acid is metered into the potable water in a concentration in the range from 2 to 2000 mg/l.

4. The method as claimed in any one of claims 1 to 3, **characterized in that** low molecular weight hyaluronic acid with a molecular weight <400 000 Daltons or high molecular weight hyaluronic acid with a molecular weight >1 000 000 Daltons is metered into the potable water.

5. The method as claimed in any one of claims 1 to 4, **characterized in that** the hyaluronic acid is admixed with a preservative, in particular sorbic acid.

6. A device for providing a composition containing hyaluronic acid, in particular for cosmetic or medical purposes, comprising a metering device (10, 30, 50) containing hyaluronic acid, **characterized in that** the metering device (10, 30, 50) has a pressure connection (58) for introducing potable water under pressure from a potable water network (20), wherein the composition from potable water and hyaluronic acid is formed by means of the metering device (10, 30, 50) and is discharged via a showerhead (28) of a shower (16) for use on the skin.

7. The device as claimed in claim 6, **characterized in that** the metering device (10, 30, 50) has an interchangeable cartridge (68) containing hyaluronic acid.

8. The device as claimed in claim 7, **characterized in that** a non-return valve (62, 64) is arranged in the inlet and outlet of the interchangeable cartridge (68).

9. The device as claimed in any one of claims 6 to 8, **characterized in that** the metering device (10, 30, 50) has a metering pump (14) for metering liquid or a metering lock (52) for metering solids.

10. The device as claimed in any one of claims 6 to 9, **characterized in that** the metering device (10, 30, 50) is switchable via a reversing valve (54) as required for supplying the composition or potable water.

## Revendications

1. Procédé permettant de fournir une composition contenant de l'acide hyaluronique destinée notamment à des fins cosmétiques ou médicales, dans lequel une réserve d'acide hyaluronique est constituée dans un dispositif de dosage (10, 30, 50), **caractérisé en ce que** de l'eau potable sous pression, issue d'un réseau de distribution d'eau potable (20), est introduite dans le dispositif de dosage (10, 30, 50) à travers une conduite sous pression et enrichie en acide hyaluronique au moyen du dispositif de dosage (10, 30, 50) pour ainsi former ladite composition, et que la composition formée à partir d'eau potable et d'acide hyaluronique est distribuée à travers un pommeau (28) d'une douche (16) pour être appliquée sur la peau.

2. Procédé selon la revendication 1, **caractérisé en ce que** l'on fait passer ladite eau potable par le dispositif de dosage (10, 30, 50) pour que celle-ci absorbe de l'acide hyaluronique.

3. Procédé selon les revendications 1 ou 2, **caractérisé en ce que** de l'acide hyaluronique est introduit dans ladite eau potable de manière à ce que sa concentration soit comprise entre 2 et 2 000 mg/L.

4. Procédé selon l'une des revendications 1 à 3, **caractérisé en ce que** de l'acide hyaluronique à faible masse moléculaire ayant une masse moléculaire < 400 000 Dalton ou de l'acide hyaluronique à haute masse moléculaire ayant une masse moléculaire supérieure à 1 000 000 Dalton est introduit dans ladite eau potable.

5. Procédé selon l'une des revendications 1 à 4, **caractérisé en ce qu'**un agent conservateur, notamment de l'acide sorbique, est ajouté audit acide hyaluronique.

6. Dispositif permettant de fournir une composition contenant de l'acide hyaluronique destinée notamment à des fins cosmétiques ou médicales, lequel comprend un dispositif de dosage (10, 30, 50) contenant de l'acide hyaluronique, **caractérisé en ce que** le dispositif de dosage (10, 30, 50) comporte un raccordement sous pression (58) permettant d'introduire de l'eau potable sous pression issue d'un réseau de distribution d'eau potable (20), la composition étant formée au moyen du dispositif de dosage (10, 30, 50) à partir d'eau potable et d'acide hyaluronique puis distribuée à travers un pommeau (28) d'une douche (16) pour être appliquée sur la peau.

7. Dispositif selon la revendication 6, **caractérisé en ce que** le dispositif de dosage (10, 30, 50) comporte une cartouche échangeable (68) contenant de l'acide hyaluronique.

8. Dispositif selon la revendication 7, **caractérisé en ce que** des clapets anti-retour (62, 64) sont disposés en amont et en aval de la cartouche échangeable (68).

9. Dispositif selon l'une des revendications 6 à 8, **caractérisé en ce que** le dispositif de dosage (10, 30, 50) comporte une pompe de dosage (14) permettant d'introduire un liquide ou un sas de dosage (52) permettant d'introduire un solide.

10. Dispositif selon l'une des revendications 6 à 9, **caractérisé en ce qu'**une vanne de commutation (54) permet de commuter le dispositif de dosage (10, 30, 50) de manière à ce qu'il fournisse, selon les besoins, ladite composition ou de l'eau potable.
